# EUROPEAN PATENT SPECIFICATION

(11) **EP 2 301 618 B1**
(45) Date of publication and mention of the grant of the patent: **07.12.2011**
(21) Application number: 09425369.7
(22) Date of filing: 23.09.2009
(51) Int. Cl.: A61M 25/06

(54) **A dilation device for dilating an opening**
Dilatationsvorrichtung zum Dilatieren einer Öffnung
Dispositif de dilatation pour la dilatation d'une ouverture

(43) Date of publication of application: 30.03.2011
(73) Proprietor: Covidien AG, 8212 Neuhausen am Rheinfall (CH)
(72) Inventor: Resca, Daniele, 41038 San Felice sul Panaro (Modena) (IT); Zucchi, Giuseppe, 41039 Possidonio (Modena) (IT)
(74) Representative: Lanzoni, Luciano

(56) References cited:
- WO-A2-03/057272
- WO-A2-2008/024883
- US-A1- 2007 255 305
- US-A1- 2008 177 249

## Description

This invention refers to a dilation device for dilating an opening.

In particular, this invention refers to a device designed to dilate an entry to a human/animal body part, system or tract (i.e. respiratory system, digestive system, intestinal apparatus, urinary tract); the entry or opening, can be natural or artificial, the latter being made through a percutaneous technique to form a stoma.

Even more particularly, this invention refers to a dilation device for use during tracheostomy procedures to dilate a stoma in a trachea, allowing the positioning of a tracheostomy tube or a catheter.

In the respiratory field, when a patient has pathological conditions or injury preventing its normal respiratory functioning (i.e. reduced patency of upper airways), or shall undergo to mechanical ventilation for a prolonged time, it is necessary to perform a tracheostomy thus allowing better management and control of patient ventilation.

In addition to the traditional techniques used to perform a tracheotomy, those currently employed are classified as percutaneous techniques when, after an initial incision and partial insertion of a needle in the required position, various instruments are used in order to dilate the stoma for the final positioning of the tracheostomy tube. The instruments currently used comprise kits for percutaneous tracheostomy consisting of a series of rigid dilators with tapered distal ends.

The dilators have different dimensions depending on the size of the cannula to be inserted.

The disadvantage of using such instruments is the possible damage that can be caused to the patient's trachea, as a result of the compression force exerted by the operator while inserting the dilator. The exerted force is partially transferred to the circumference of the stoma, but most of the force is erroneously transferred axially to the front wall of the trachea, and may even cause rupture of the trachea rings.

There is also a high risk of injuring the rear wall of the trachea, because of the difficulty of stopping to push the dilator once the trachea front wall succumb to the dilation.

Further damage to the tissue around the stoma is caused by the friction between the dilator surface and the tissues, especially during a pushing manoeuvre where the tissues offers a very high resistance to penetration.

For example, such a disadvantage is observed in document US 2007255305, which shows a percutaneous dilation apparatus. Such an apparatus includes an elongate dilation tube, a plurality of elongate expansion members disposed in a nested concentric arrangement aground the elongate expansion tube, and an outer sheath for capturing the plurality of elongate expansion members and maintaining the apparatus in an assembled configuration. Each expansion member is independently movable from a first to a second position along the central axis of the tube as well as each concentrically smaller member. Each expansion member presents a tapered end that penetrates into the stoma and that makes friction with the tissue of the stoma.

The difference in resistance of the tissues and the friction caused between the edge of the stoma and the outer wall of the rigid dilator can make it difficult to obtain a circular stoma cross-section. In this setting, the technical task on which this invention as claimed in claim 1 is based is to provide a dilation device for dilating an opening which overcomes the prior art drawbacks described above.

In particular, the aim of this invention as claimed in claim 1 is to provide a dilation device for dilating an opening which can reduce the risk of damage to tissues and internal body parts. Even more particularly the aim of this invention as claimed in claim 1 is to provide a dilation device for performing a tracheostomy, which can reduce the risk of damage to the patient's trachea and surrounding tissues.

A further aim of the invention as claimed in claim 1 is to provide a dilation device for dilating an opening which reduces damage to the: tissues caused by the friction produced between the edges of the stoma and the outer surface of the device once the dilator is pushed to penetrate the entry to dilate the stoma.

Finally, the aim of this invention as claimed in claim 1 is to provide a dilation device for dilating an opening which is practical and easy to use.

The technical task and the aims described above are substantially achieved by a dilation device for dilating an opening comprising the technical characteristics described in one or more of the accompanying claims.

Additional characteristics and advantages of this invention will become clearer from the non-binding description of a preferred but not exclusive embodiment of a dilation device for dilating an opening, as illustrated in the accompanying drawings in which:
- figure 1 is a cross-section view of a first embodiment of the dilation device according to the invention;
- figure 1a is a plan projection of figure 1 cross-sectioned along the line A-A;
- figures 2 and 3 are cross-section views of the device in figure 1 in two different stages of use;
- figures 4-7 are cross-section views of the individual components of the device according to the invention, shown in assembled form in figure 1;
- figures 4a-6a are plan projections of figures 4-6 cross-sectioned along the line A-A;
- figures 8-11 show four different stages of use of a second embodiment of the dilation device according to the invention.

With reference to the accompanying drawings, a dilation device 1 as claimed in claim 1 for dilating an opening according to this invention comprises an axially hollow flexible tubular body 2 with a tapered distal end 2a.

The end 2a is conical in order to define a guide for the insertion of the device 1 into a percutaneous incision made in the patient's trachea.

The tubular body 2 comprises a plurality of coaxial and axially hollow cylindrical elements 3, 4, 5 and 6, each having a respective first tapered end 3a, 4a, 5a and 6a.

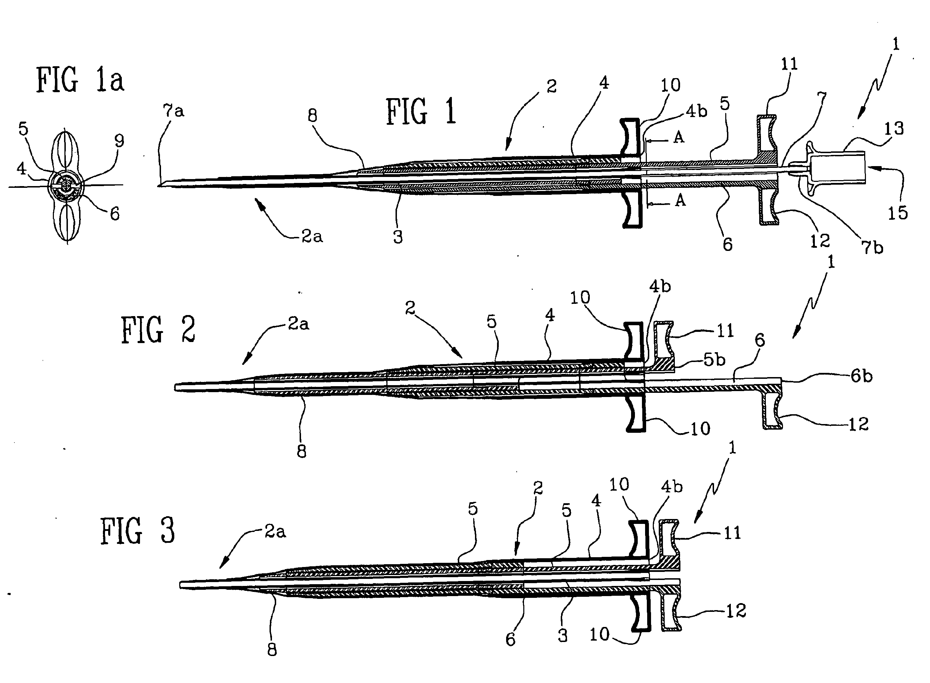

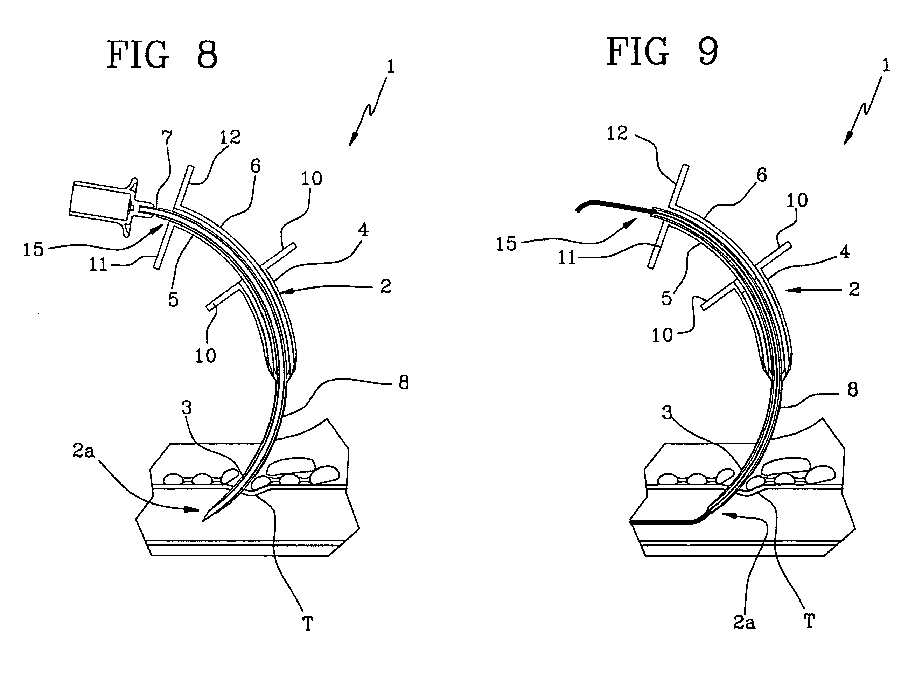

the tubular body 2 is made from flexible material and can present a rectilinear axis configuration as shown in figures 1-3, or a curved axis configuration as shown in figures 8-11. The tubular body 2 is preferably made from biocompatible, antimicrobial or bio-static (inhibiting bacterial and fungi growth), soft and flexible plastic material.

at least one of the coaxial cylindrical elements 3-6 is slidably arranged and movable along the axis towards the tapered end 2a of the tubular body 2.

The tapered end of the sliding cylindrical element thus approaches the end 2a already inserted in the stoma or opening, gradually penetrating and retracting the stoma without excessive trauma for the patient.

With reference to figures 1 and 4-7, the coaxial cylindrical elements of the tubular body 2 comprise a central inner cannula 3, a cylindrical outer sleeve 4 and at least one cylindrical dilator 5, positioned between the central, inner cannula 3 and the sleeve 4, sliding axially on the central, inner cannula 3.

The central, inner cannula 3 presents a first tapered end 3a which substantially constitutes the largest portion of the end 2a of the tubular body 2.

The outer sleeve 4 presents a first end 4a connected to the first end 3a of the central, inner cannula 3 by means of a flexible membrane 8.

The outer sleeve 4 and the central inner cannula 3 are preferably fixed axially to each other and are connected together also at their respective second ends 3b and 4b, opposite to the first ends 3a and 4a.

In particular, the second end 4b of the sleeve 4 presents a diametrical blocking element 9 which connects the second end 3b of the central, inner cannula 3 with the edges of the second end 4b of the sleeve 4. This blocking element 9 is, for example, a diametrical segment of the same material as the sleeve 4 and the central, inner cannula 3.

In addition, at its second end 4b, the sleeve 4 presents a gripping and maneuvering device 10.

Advantageously, at least two cylindrical dilators 5, 6 are positioned between the central, inner cannula 3 and the outer sleeve 4. Merely as non-binding examples, the accompanying figures show devices with only two intermediate dilators 5 and 6. Configurations not shown can be equipped with a greater number of dilators.

Each intermediate cylindrical dilator 5, 6 presents a respective first distal tapered end 5a, 6a to allow the gradual insertion of each dilator 5, 6 into the stoma, progressively and continuously retracting it without trauma for the patient.

Preferably, the device 1 according to the invention also comprises a surgical instrument 7. In detail, the central inner cannula 3 is an axially hollow cylindrical tube, inside which other surgical instruments 15 can be inserted, such as needles 7 or guide wires 14.

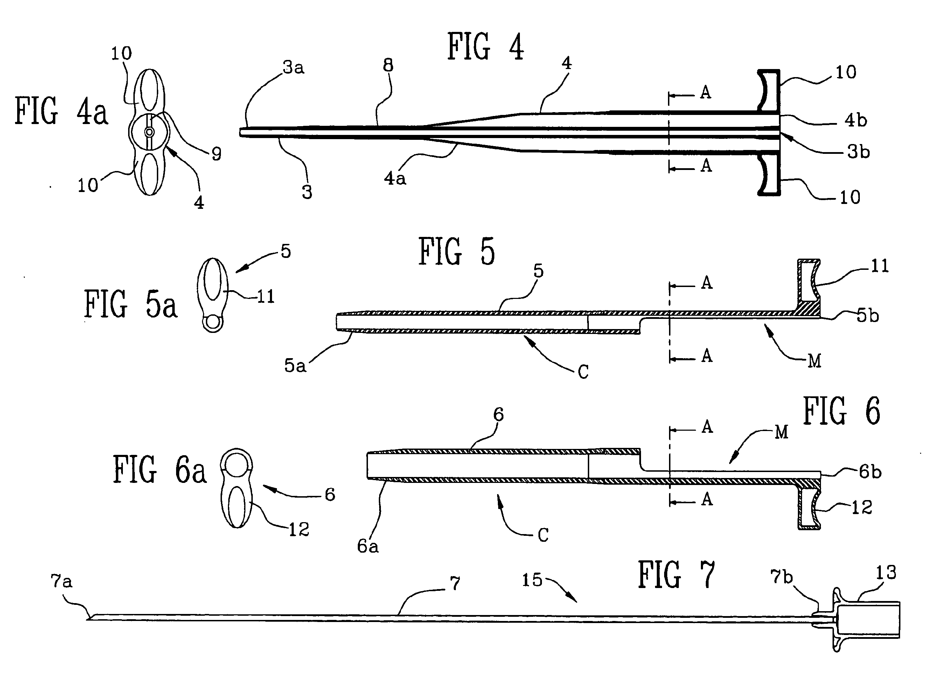

Advantageously, a surgical instrument 15, which can be inserted in the cannula 3, can be, as shown in figures 1 and 7, a rod 7 designed to make an incision and, at its first end 7a, it thus presents a needle or cutting tip. At its second end 7b, opposite to the first one, the cutting instrument or rod 7 presents a grip 13.

Once the incision has been made and the tapered end 3a of the central, inner cannula 3 has been inserted in the stoma, the needle is pulled out of the central, inner cannula 3 and the stoma is dilated. In particular, one dilator 5, 6 at a time is moved towards the end 2a of the tubular body 2. The individual dilators 5, 6 move independently of each other. They move telescopically, sliding axially with respect to each other, until they at least partially overlap the first tapered end 3a of the central, inner cannula 3.

The cylindrical elements 3-6 present different axial lengths.

In particular, the central, inner cannula 3 has a greater axial length than the other cylindrical elements.

The intermediate cylindrical dilators 5, 6 present gradually shorter lengths as their external diameters increase.

Finally, the outer sleeve 4 presents a shorter axial length than all the cylindrical elements forming the tubular body 2.

The cylindrical dilators 5, 6 each present a respective second end 5b and 6b, opposite the first end 5a, 6a, where a gripping and maneuvering device 11 and 12 is positioned.

As shown in figures 5 and 6, each cylindrical dilator 5, 6 preferably comprises a main cylindrical hollow body C presenting a tapered end and a handle M terminating with a respective gripping device 11, 12.

As described above, the cylindrical elements are coaxial and inserted one inside the other.

The terminal elements are represented by the central, inner cannula 3 and the sleeve 4.

The intermediate cylindrical dilators 5, 6 thus present diameters which vary between the diameter of the central, inner cannula 3 and the diameter of the sleeve 4.

When in use, after inserting in the incision the tapered end 3a of the central, inner cannula 3, the dilators 5, 6 are pushed one after the other towards the tapered end 3a of the central, inner cannula 3, taking care to move the dilators in order, from the one with the smallest diameter and the greatest axial length to the outermost one with the largest diameter and the shortest axial length.

The dilators 5, 6 are moved by operating the gripping and maneuvering device 11, 12 and pushed forward in sequence independently of each other.

The innermost dilator 5 is pushed first and slides in the central, inner cannula 3, inside the sleeve 4, gradually dilating the membrane 8 connecting the central, inner cannula 3 and the sleeve 4.

The second dilator 6, with a slightly larger diameter than the first, is then pushed forward. The second dilator 6 thus slides over the first one 5.

In the presence of two or more dilators, this operation is repeated until all the dilators are close to the tapered end 2a of the tubular body 2.

Since the dilators have progressively larger diameters, their advancement in sequence towards the end 2a of the tubular body 2 causes the gradual dilation of the membrane 8 and thus of the stoma.

The presence of the membrane 8 reduces the friction between the outer walls of the device and the tissue at the edges of the stoma, so as to minimise the patient's suffering.

Similarly, when the device has to be removed from the patient's trachea, the operator withdraws one dilator at a time in sequence, generating the contraction of the membrane 8, while the walls of the stoma remains dilated for a sufficient time to allow insertion of the tracheostomy tube. To limit the patient's suffering, and speed the extraction manoeuvre the outermost dilator, with the largest diameter, is removed first.

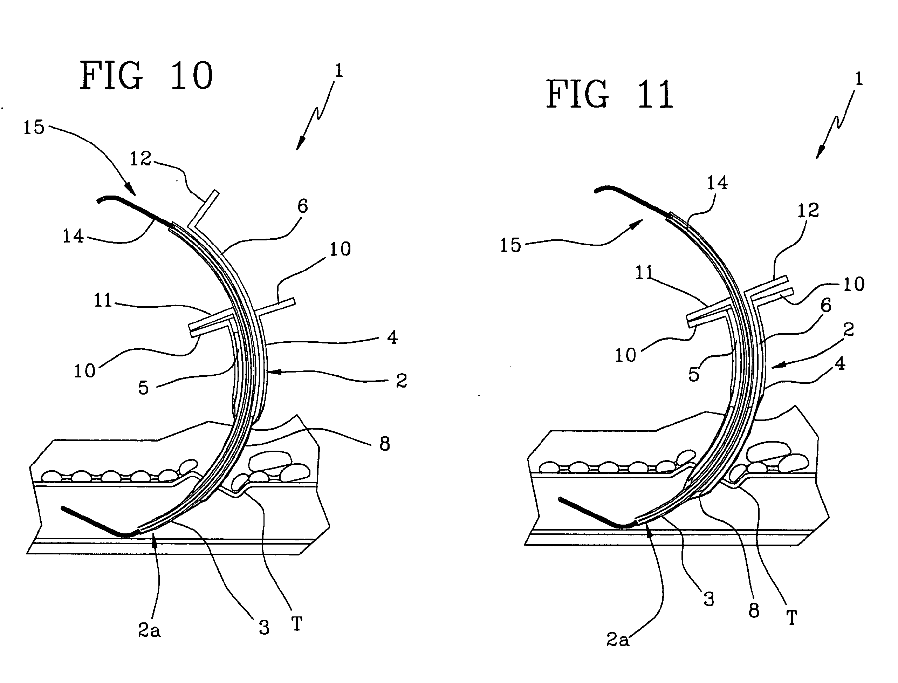

During insertion of the device, the travel of the dilators is limited by the interference of the respective gripping and maneuvering device 11 and 12 with the gripping and maneuvering device 10 of the sleeve 4, as can be seen in figures 3 and 11.

When the dilators 5, 6 are withdrawn from the stoma, on the other hand, their travel is limited by the diametrical blocking element 9, present at the second end 4b of the outer sleeve 4, acting as a stop. This prevents the dilators 5, 6 from slipping out of their housing.

This invention achieves the proposed aims and provides important advantages.

The dilation effect is achieved by means of a series of expansion components inserted one after the other to obtain calibrated mechanical dilation.

The gradual insertion of the dilators inside the stoma make it possible, in fact, to exert equally distributed radial forces on all the tissue surrounding the stoma.

There are therefore no axial forces that can damage the trachea.

The device is intrinsically secure since once the metal needle used to make the stoma is withdrawn, the tip of the dilator, being soft and flexible, cannot damage the rear wall of the trachea if it comes into contact with it.

A further advantage of this invention is the fact that once achieved complete dilation with the device, the created stoma has the suitable size to receive inside of it the cannula of the desired size; in this way, the device according to the invention allows a calibrated dilation and never an over-dilation or under-dilation with respect to the one desired.

Accordingly, the device of the present invention can be provided in a variety of sizes to serve, for example, all tracheostomy cannula sizes commercially available.

## Claims

1. A dilation device for dilating an opening into a human/animal body part comprising a flexible axially hollow tubular body (2) with a tapered end (2a), forming a guide, which can be inserted in the opening; the tubular body (2) comprising a plurality of coaxial cylindrical elements (3, 4, 5, 6) each of which having at least a first tapered end (3a, 4a, 5a, 6a); at least one of these coaxial cylindrical elements (3, 4, 5, 6) being slidably arranged and movable towards the tapered end (2a) of the tubular body (2) to gradually dilate the opening; the plurality of coaxial cylindrical elements (3, 4, 5, 6) comprising a central inner cannula (3), an outer cylindrical sleeve (4) and at least one cylindrical dilator 5, positioned between the central inner cannula 3 and the sleeve 4; the device being **characterised in that** the outer sleeve (4) is connected to the central inner cannula (3) by means of a flexible membrane (8) connecting the first end (4a) of the sleeve (4) with the first end (3a) of the central inner cannula (3).

2. A dilation device according to claim 1, **characterised in that** the at least one intermediate cylindrical dilator (5, 6) is slidably arranged and movable axially on the central inner cannula (3) to advance its own tapered end (5a, 6a) close to the first tapered end (3a) of the central inner cannula (3).

3. A dilation device according to claim 2, **characterised in that** it comprises at least two intermediate cylindrical dilators (5, 6), each of which being slidably arranged and movable axially and independently of each other towards the first tapered end (3a) of the central inner cannula (3), to gradually dilate the entry in sequence.

4. A dilation device according to claim 1, **characterised in that** the sleeve (4) is connected to the central inner cannula (3) at their respective second ends (4b, 3b), opposite to the first ends (4a, 3a), by means of a blocking element (9) which extends diametrically at the second end (4b) of the sleeve (4).

5. A dilation device according to claim 4, **characterised in that** the blocking element (9) keeps the intermediate cylindrical dilators (5, 6) inside the sleeve (4), preventing them from slipping out of the sleeve (4).

6. A dilation device according to any of the claims from 3 to 5, **characterised in that** each intermediate cylindrical dilator (5, 6) presents a second end (5b, 6b) opposite the first one (5a, 6a), and a gripping and maneuvering device (11, 12); the intermediate cylindrical dilators (5, 6) being movable one at a time towards the tapered end (2a) of the tubular body (2), and away from it.

7. A dilation device according to any of the claims from 3 to 6, **characterised in that** the intermediate cylindrical dilators (5, 6) have different lengths.

8. A dilation device according to any of the foregoing claims, **characterised in that** the sleeve (4) at the second end (4b), opposite the first (4a), presents a gripping and maneuvering device (10).

9. A dilation device according to any of the foregoing claims, **characterised in that** it comprises a surgical instrument (15) which can be inserted in the central inner cannula (3).

10. A dilation device according to claim 9, **characterised in that** the instrument (15) is a rod (7) presenting at its first end (7a) a needle designed to cut a body part to enter a body; the cutting rod (7) being withdrawable from the central inner cannula (3) once the incision has been made.

11. A dilation device according to any of the foregoing claims, **characterised in that** the flexible tubular body (2) has a rectilinear axis.

12. A dilation device according to any of the foregoing claims from 1 to 10, **characterised in that** the flexible tubular body (2) has a curved axis.

## Patentansprüche

1. Dilatationsvorrichtung zum Dilatieren einer Öffnung in einem menschlichen oder tierischen Körperteil, umfassend einen flexiblen, axial hohlen, röhrenförmigen Körper (2) mit einem konischen Ende (2a), der eine Führung bildet, der in die Öffnung eingeführt werden kann, wobei der röhrenförmige Körper (2) eine Vielzahl koaxialer zylindrischer Elemente (3, 4, 5, 6) umfasst, von denen jedes mindestens ein erstes konisches Ende (3a, 4a, 5a, 6a) aufweist, wobei mindestens eines dieser koaxialen zylindrischen Elemente (3, 4, 5, 6) verschiebbar angeordnet und in Richtung des konischen Endes (2a) des röhrenförmigen Körpers (2) beweglich ist, um die Öffnung schrittweise zu dilatieren, wobei die Vielzahl koaxialer zylindrischer Elemente (3, 4, 5, 6) eine zentrale innere Kanüle (3), eine äußere zylindrische Hülse (4) und mindestens einen zylindrischen Dilatator (5) umfasst, der zwischen der zentralen inneren Kanüle (3) und der Hülse (4) positioniert ist, wobei die Vorrichtung **dadurch gekennzeichnet ist, dass** die äußere Hülse (4) mit der zentralen inneren Kanüle (3) mithilfe einer flexiblen Membran (8) verbunden ist, welche das erste Ende (4a) der Hülse (4) mit dem ersten Ende (3a) der zentralen inneren Kanüle (3) verbindet.

2. Dilatationsvorrichtung nach Anspruch 1, **dadurch gekennzeichnet, dass** der mindestens eine dazwischenliegende zylindrische Dilatator (5, 6) auf der zentralen inneren Kanüle (3) verschiebbar angeordnet und axial beweglich ist, um sein konisches Ende (5a, 6a) nah an das erste konische Ende (3a) der zentralen inneren Kanüle (3) vorzurücken.

3. Dilatationsvorrichtung nach Anspruch 2, **dadurch gekennzeichnet, dass** sie mindestens zwei dazwischenliegende zylindrische Dilatatoren (5, 6) umfasst, von denen jeder verschiebbar angeordnet und axial beweglich und unabhängig voneinander in Richtung des ersten konischen Endes (3a) der zentralen inneren Kanüle (3) beweglich ist, um den Eingang nacheinander schrittweise zu dilatieren.

4. Dilatationsvorrichtung nach Anspruch 1, **dadurch gekennzeichnet, dass** die Hülse (4) mit der zentralen inneren Kanüle (3) an ihren jeweiligen zweiten Enden (4b, 3b) gegenüber den ersten Enden (4a, 3a) mithilfe eines Blockierelements (9) verbunden ist, das sich diametral am zweiten Ende (4b) der Hülse (4) erstreckt.

5. Dilatationsvorrichtung nach Anspruch 4, **dadurch gekennzeichnet, dass** das Blockierelement (9) die dazwischenliegenden zylindrischen Dilatatoren (5, 6) innerhalb der Hülse (4) hält, indem es verhindert, dass sie aus der Hülse (4) herausrutschen.

6. Dilatationsvorrichtung nach einem der Ansprüche 3 bis 5, **dadurch gekennzeichnet, dass** jeder dazwischenliegende zylindrische Dilatator (5, 6) ein zweites Ende (5b, 6b) gegenüber dem ersten (5a, 6a) sowie eine Greif- und Steuerungsvorrichtung (11, 12) aufweist, wobei jeweils einer der dazwischenliegenden zylindrischen Dilatatoren (5, 6) in Richtung des konischen Endes (2a) des röhrenförmigen Körpers (2) und davon weg beweglich ist.

7. Dilatationsvorrichtung nach einem der Ansprüche 3 bis 6, **dadurch gekennzeichnet, dass** die dazwischenliegenden zylindrischen Dilatatoren (5, 6) unterschiedliche Längen aufweisen.

8. Dilatationsvorrichtung nach einem der vorangehenden Ansprüche, **dadurch gekennzeichnet, dass** die Hülse (4) am zweiten Ende (4b) gegenüber dem ersten (4a) eine Greif- und Steuerungsvorrichtung (10) aufweist.

9. Dilatationsvorrichtung nach einem der vorangehenden Ansprüche, **dadurch gekennzeichnet, dass** sie ein chirurgisches Instrument (15) aufweist, dass in die zentrale innere Kanüle (3) eingeführt werden kann.

10. Dilatationsvorrichtung nach Anspruch 9, **dadurch gekennzeichnet, dass** das Instrument (15) ein Stäbchen (7) ist, das an seinem ersten Ende (7a) eine Nadel aufweist, die dazu konzipiert ist, an einem Körperteil einen Schnitt vorzunehmen, um in einen Körper zu gelangen, wobei das Schneidestäbchen (7) aus der zentralen inneren Kanüle (3) herausziehbar ist, wenn die Inzision erfolgt ist.

11. Dilatationsvorrichtung nach einem der vorangehenden Ansprüche, **dadurch gekennzeichnet, dass** der flexible röhrenförmige Körper (2) eine gerade Achse aufweist.

12. Dilatationsvorrichtung nach einem der vorangehenden Ansprüche 1 bis 10, **dadurch gekennzeichnet, dass** der flexible röhrenförmige Körper (2) eine krumme Achse aufweist.

## Revendications

1. Dispositif de dilatation pour la dilatation d'une ouverture dans une partie du corps humain/animal comprenant un corps tubulaire souple axialement creux (2) avec une extrémité conique (2a), formant un guide, qui peut être introduit dans l'ouverture ; le corps tubulaire (2) comprenant une pluralité d'éléments cylindriques coaxiaux (3, 4, 5, 6), ayant chacun au moins une première extrémité conique (3a, 4a, 5a, 6a) ; au moins l'un de ces éléments cylindriques coaxiaux (3, 4, 5, 6) étant disposés de façon coulissante et pouvant se déplacer en direction de l'extrémité conique (2a) du corps tubulaire (2) pour dilater graduellement l'ouverture ; la pluralité d'éléments cylindriques coaxiaux (3, 4, 5, 6) comprenant une canule interne centrale (3), un manchon cylindrique externe (4) et au moins un dilatateur cylindrique (5), positionné entre la canule interne centrale (3) et le manchon (4) ; le dispositif **se caractérisant en ce que** le manchon externe (4) est raccordé à la canule interne centrale (3) au moyen d'une membrane souple (8) raccordant la première extrémité (4a) du manchon (4) à la première extrémité (3a) de la canule interne centrale (3).

2. Dispositif de dilatation selon la revendication 1, **caractérisé en ce que** le au moins un dilatateur cylindrique intermédiaire (5, 6) est disposé de façon coulissante et peut se déplacer axialement sur la canule interne centrale (3) pour rapprocher sa propre extrémité conique (5a, 6a) de la première extrémité conique (3a) de la canule interne centrale (3).

3. Dispositif de dilatation selon la revendication 2, **caractérisé en ce qu'**il comprend au moins deux dilatateurs cylindriques intermédiaires (5, 6), étant chacun disposé de façon coulissante et pouvant se déplacer, axialement et indépendamment l'un de l'autre, en direction de la première extrémité conique (3a) de la canule interne centrale (3), pour dilater graduellement en séquence l'entrée.

4. Dispositif de dilatation selon la revendication 1, **caractérisé en ce que** le manchon (4) est raccordé à la canule interne centrale (3), au niveau de ses secondes extrémités respectives (4b, 3b), en face des premières extrémités (4a, 3a), au moyen d'un élément de blocage (9) qui évolue diamétralement au niveau de la seconde extrémité (4b) du manchon (4).

5. Dispositif de dilatation selon la revendication 4 **caractérisé en ce que** l'élément de blocage (9) garde les dilatateurs cylindriques intermédiaires (5, 6) à l'intérieur du manchon (4), pour les empêcher de glisser à l'extérieur du manchon (4).

6. Dispositif de dilatation selon l'une quelconque des revendications 3 à 5, **caractérisé en ce que** chaque dilatateur cylindrique intermédiaire (5, 6) présente une seconde extrémité (5b, 6b) en face de la première (5a, 6a), et un dispositif de préhension et de manutention (11, 12) ; les dilatateurs cylindriques intermédiaires (5, 6) pouvant se rapprocher, un à la fois, de l'extrémité conique (2a) du corps tubulaire (2), et s'en éloigner.

7. Dispositif de dilatation selon l'une quelconque des revendications 3 à 6, **caractérisé en ce que** les dilatateurs cylindriques intermédiaires (5, 6) ont des longueurs différentes.

8. Dispositif de dilatation selon l'une quelconque des revendications précédentes, **caractérisé en ce que** le manchon (4), au niveau de la seconde extrémité (4b), en face de la première (4a), présente un dispositif de préhension et de manutention (10).

9. Dispositif de dilatation selon l'une quelconque des revendications précédentes, **caractérisé en ce qu'**il comprend un instrument de chirurgie (15) qui peut être introduit dans la canule interne centrale (3).

10. Dispositif de dilatation selon la revendication 9, **caractérisé en ce que** l'instrument (15) est une tige (7) présentant, au niveau de sa première extrémité (7a), une aiguille conçue pour pratiquer une incision dans une partie du corps afin de pénétrer dans un corps ; la tige tranchante (7) pouvant être retirée de la canule interne centrale (3) une fois l'incision effectuée.

11. Dispositif de dilatation selon l'une quelconque des revendications précédentes, **caractérisé en ce que** le corps tubulaire souple (2) présente un axe rectiligne.

12. Dispositif de dilatation selon l'une quelconque des revendications précédentes 1 à 10, **caractérisé en ce que** le corps tubulaire souple (2) présente un axe curviligne.
